# FASCICULE DE BREVET EUROPEEN

(11) **EP 2 708 604 B1**
(45) Date de publication et mention de la délivrance du brevet: **26.04.2017**
(21) Numéro de dépôt: 13184238.7
(22) Date de dépôt: 13.09.2013
(51) Int. Cl.: C12Q 1/04, G01N 33/00

(54) **Procédé de détermination d'un risque de contamination aspergillaire basé sur la detection de composés organiques volatiles microbiens**
Verfahren zur Bestimmung ein Kontaminierungsgefahr durch Aspergillus basierend auf dem Nachweis von mikrobiellen flüchtigen organischen Substanzen
Method for determining a risk of aspergillus contamination based on the detection of microbial volatile organic compounds

(30) Priorité: 14.09.2012 FR 1258646
(43) Date de publication de la demande: 19.03.2014
(73) Titulaire: Centre Scientifique Et Technique Du Batiment (CSTB), 77420 Champs sur Marne (FR)
(72) Inventeur: Moularat, Stéphane, 77185 Lagnes (FR); Robine, Enric, 77400 Lagny-sur-Marne (FR)
(74) Mandataire: Chaffraix, Jean

(56) Documents cités:
- Yaël Joblin: "Elaboration d'un microsystème d'analyse de l'air destiné à la détection rapide d'un développement fongique dans les espaces clos", , 13 mai 2011 (2011-05-13), pages 30-73, XP055052996, Extrait de l'Internet: URL:http://tel.archives-ouvertes.fr/docs/0 0/69/66/18/PDF/TH2011PEST1027_complete.pdf [extrait le 2013-02-11]
- FISCHER G ET AL: "Species-specific production of microbial volatile organic compounds (MVOC) by airborne fungi from a compost facility", CHEMOSPHERE, PERGAMON PRESS, OXFORD, GB, vol. 39, no. 5, 1 août 1999 (1999-08-01), pages 795-810, XP002345600, ISSN: 0045-6535, DOI: 10.1016/S0045-6535(99)00127-7
- VIVIANA POLIZZI ET AL: "Identification of volatile markers for indoor fungal growth and chemotaxonomic classification of Aspergillus species", FUNGAL BIOLOGY, vol. 116, no. 9, 29 juin 2012 (2012-06-29) , pages 941-953, XP055052968, DOI: 10.1016/j.funbio.2012.06.001

## Description

### DOMAINE TECHNIQUE DE L'INVENTION

La présente invention concerne un procédé de détermination d'une empreinte chimique spécifique d'une contamination aspergillaire dans des environnements intérieurs.

Par environnement intérieur on entend un espace confiné à l'intérieur d'un bâtiment qui est aéré de façon non continue. Des exemples d'environnements intérieurs peuvent être trouvés dans les habitations, les musées, les églises, les caves, les monuments historiques, les bâtiments administratifs, les écoles et les hôpitaux.

Le développement fongique s'accompagne de l'émission de COVm (Composés Organiques Volatils d'origine microbienne) dès le début de leur développement et durant l'ensemble des phases de croissance des micromycètes.

### ETAT DE LA TECHNIQUE ANTERIEURE

Dans ce domaine, on connaît la demande WO 2004/051226 qui porte sur des procédés de surveillance d'environnements à risque par rapport à la présence ou à l'absence de microbes. Cette invention se caractérise par la recherche d'un marqueur cnp60.

Cependant, ce document se limite à la recherche d'une chaperonine (cnp60) ce qui implique des extractions cellulaires qui doivent être sélectives de cette chaperonine.

Ce type de procédé implique une croissance significative de micromycètes, puis une collection et une extraction de la chaperonine. Bien entendu, il est préférable d'éviter la génération de micromycètes potentiellement dangereuses pour l'être humain. En outre une détection de chaperonine s'avère très difficile en comparaison avec une détection de COV.

Pour pallier à ces inconvénients, la demanderesse a mis au point un procédé de détection d'une contamination fongique d'un environnement intérieur, à l'aide du calcul d'un indice chimique de contamination fongique. Ce procédé est décrit dans la demande WO 2008/125770. Cet outil a permis de statuer sur la présence de micromycètes dans 37 à 42% des logements français à l'occasion de la campagne nationale de l'Observatoire de la Qualité de l'Air intérieur (Moularat et al., 2008a).

Cependant ce procédé ne permet pas spécifiquement de conclure s'il y a ou non un risque de contamination aspergillaire.

Polizzi et al. (Fungal Biology, 116, 941-953, 2012) ont décrit la détection de différents COVm dans des locaux contaminés par des micromycètes, et la comparaison des COVm détectés avec les profils de COVm produits par les micromycètes isolés de ces mêmes locaux, dont plusieurs espèces d'Aspergillus, lorsqu'ils sont cultivés in vitro sur milieu MEA (Malt extract agar). Ces auteurs rapportent que 4 COVm (α-copaène, géranyl acétone, trans-calaménène, and α-calacorène), détectés dans des locaux où la présence d'Aspergillus est prédominante, sont également produits in vitro par certaines souches de l'espèce *Aspergillus ustus.*

### EXPOSE DE L'INVENTION

Dans ce contexte, l'indice de contamination fongique mis au point par la demanderesse et décrit dans la demande WO 2008/125770 pourrait être complété et affiné pour une détection précoce d'un développement aspergillaire.

Pour pallier aux inconvénients de l'art antérieur, la demanderesse propose un procédé de détermination d'un risque de contamination aspergillaire dans un environnement intérieur comprenant les étapes de :
(a) prélèvement d'un échantillon d'air dans l'environnement intérieur, puis
(b) détection de COVm dans l'échantillon, cette étape comprenant une recherche d'une empreinte chimique comprenant au moins une molécule cible qui est un COVm, associé à un métabolisme aspergillaire, ladite molécule cible étant sélectionnée parmi les COVm suivants : 1,4-pentadiène, 4-heptanone, Diméthyldisulfide, Méthoxybenzène, 1,3-butanediol, 1,4-hexadiène, 1-méthoxy-2-méthyl-benzène, 1-octèn-3-one, 1-pentène, 2(5H)-furanone, 2-méthyl-isoboméol, 3,3-dichloro-1-propène, 3-butyn-1-ol, 3-heptanol, 3-heptanone, 3-méthyl-2-butanol, 3-méthylhexane, 4-heptanol, 4-méthyl-2-hexanone, Caryophyllène, Diméthyltrisulfide, Eremophilène, Germacrène D, Isoledène, Longifolène, Méthyl-2-éthylhexanoate, Muurolane, Terpinolène..

Par « risque de contamination aspergillaire » on entend un développement de micromycètes du genre aspergillus sur un support donné.

De façon particulièrement avantageuse, la détection de telles molécules cibles est plus facile et plus rapide que la détection de souches d'aspergillus ou de métabolites aspergillaires solubles.

Selon un mode de réalisation préféré, l'étape (b) comprend les sous-étapes suivantes avant la recherche d'une empreinte chimique comprenant au moins une molécule cible qui est un COVm, associé à un métabolisme aspergillaire :
- détection de COV dans l'échantillon, qui comprend une détection de la présence ou de l'absence de certains COV prédéterminés issus du métabolisme fongique, ces COV prédéterminés comprenant au moins un COV de chacune des trois catégories de COV suivantes :
   (1) les COV qui sont émis indépendamment de l'espèce fongique et de son support et qui ne sont émis que par des espèces fongiques ;
   (2) les COV qui sont émis indépendamment de l'espèce fongique et du support, et qui sont émis par des espèces biologiques non fongiques;
   (3) les COV qui sont émis en fonction de l'espèce fongique et/ou de son support ;
- calcul d'un indice chimique de contamination fongique en fonction respectivement de la présence et de l'absence des COV prédéfinis issus du métabolisme fongique.

Par « support » d'une espèce fongique, on entend le matériau sur lequel l'espèce fongique se développe, de préférence un matériau de construction tel que du papier peint, de la toile de verre ou autre.

Avantageusement, l'empreinte chimique est spécifique d'au moins une espèce d'aspergillus choisie parmi *Aspergillus restrictus, Aspergillus versicolor, Aspergillus sydowii, Aspergillus niger.*

En variante, ladite molécule cible est sélectionnée dans le groupe comprenant le : 1,4-pentadiène, 4-heptanone, Diméthyldisulfide, Méthoxybenzène. Ces molécules cibles sont spécifiques d'une pluralité de souches d'Aspergillus.

Selon une autre variante, ladite molécule cible est sélectionnée dans le groupe comprenant le : 1,3-butanediol, 1,4-hexadiène, 1-méthoxy-2-méthyl-benzène, 1-octèn-3-one, 1-pentène, 2(5H)-furanone, 2-méthyl-isoboméol, 3,3-dichloro-1-propène, 3-butyn-1-ol, 3-heptanol, 3-heptanone, 3-méthyl-2-butanol, 3-méthylhexane, 4-heptanol, 4-méthyl-2-hexanone, Caryophyllène, Diméthyltrisulfide, Eremophilène, Germacrène D, Isoledène, Longifolène, Méthyl-2-éthylhexanoate, Muurolane, Terpinolène. Ces molécules cibles sont spécifiques d'une ou deux souches d'Aspergillus.

Ainsi, le procédé selon l'invention peut être réalisé avec une desdites molécules cibles qui est spécifique d'une ou deux souches d'Aspergillus. En variante, cette molécule cible est spécifique de plus de deux souches d'Aspergillus.

Selon une variante préférée, l'empreinte chimique comprend au moins deux molécules cibles.

Selon une autre variante, l'empreinte chimique comprend toutes lesdites molécules cibles.

Avantageusement, le procédé comprend une étape de recherche de zones de contamination fongique réalisée avant l'étape (a). Ainsi, le procédé selon cette variante commence par cette étape.

Le procédé selon le deuxième mode de réalisation de l'invention est particulièrement utile pour la détection précoce d'un risque de contamination aspergillaire, c'est-à-dire avant l'apparition de quantités significatives pour une détection microbiologique. Cette possibilité de détection précoce est d'autant plus intéressante qu'elle ne nécessite pas une détection directe des espèces d'aspergillus. On peut ainsi conclure à la contamination aspergillaire à un stade précoce de développement des champignons. Par « stade précoce » de développement, on entend un stade où les micromycètes sont invisibles à la surface du support, et de préférence indécelables par l'analyse microbiologique de l'air, mais produisent néanmoins des métabolites et produits de dégradation inhalables et responsables dans certains cas de maladies.

### DESCRIPTION DETAILLEE D'UN MODE DE REALISATION

La présente invention repose sur l'étude en laboratoire des émissions COV de 4 espèces du genre Aspergillus :
- Aspergillus restrictus,
- A. versicolor,
- A. sydowii,
- A. niger.

Ces espèces ont été cultivées à l'abri de la lumière et à 25°C sur différents matériaux stérilisés retrouvés fréquemment contaminés dans des environnements intérieurs. Un support de référence non émissif, constitué de Fibre de verre imbibée d'une solution nutritive, a également été utilisé pour l'ensemble des souches testées. La solution nutritive utilisée est par exemple une solution aqueuse comprenant notamment du de K2HPO4, KCl, MgSO4, FeSO4, Glucose et NaNO3. Cette solution est tamponnée à un pH de 7,4.
Bien entendu, une autre solution nutritive connue peut être utilisée sans sortir du cadre de l'invention.

Le plan de manipulation est résumé dans le Tableau 1 ci-dessous.

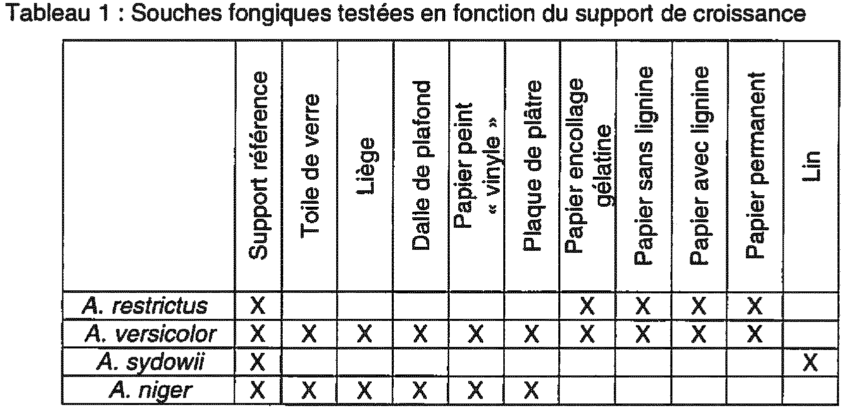

Dans le tableau 1, une croix indique l'identification de la souche d'aspergillus (en ligne) qui se développe sur le support de croissance (en colonne).

Le support de référence sert ici de témoin positif pour valider la capacité des souches d'aspergillus à se développer. Le support utilisé ici est la Fibre de verre imbibée d'une solution nutritive décrite ci-dessus. Bien entendu, un autre support de référence connu peut être utilisé.

Les souches étudiées se développent toutes sur le support de référence, et sur au moins un autre support de croissance parmi ceux testés. La souche A. sydowii ne pousse que sur du lin. Les autres souches *A. restrictus, A. versicolor, A. sydowii*, *A. niger* poussent sur plus de deux supports de croissance parmi ceux testés.

Lors de cette étude, 28 COV ont été identifiés uniquement à partir de souches d'Aspergillus (Tableau 2) :

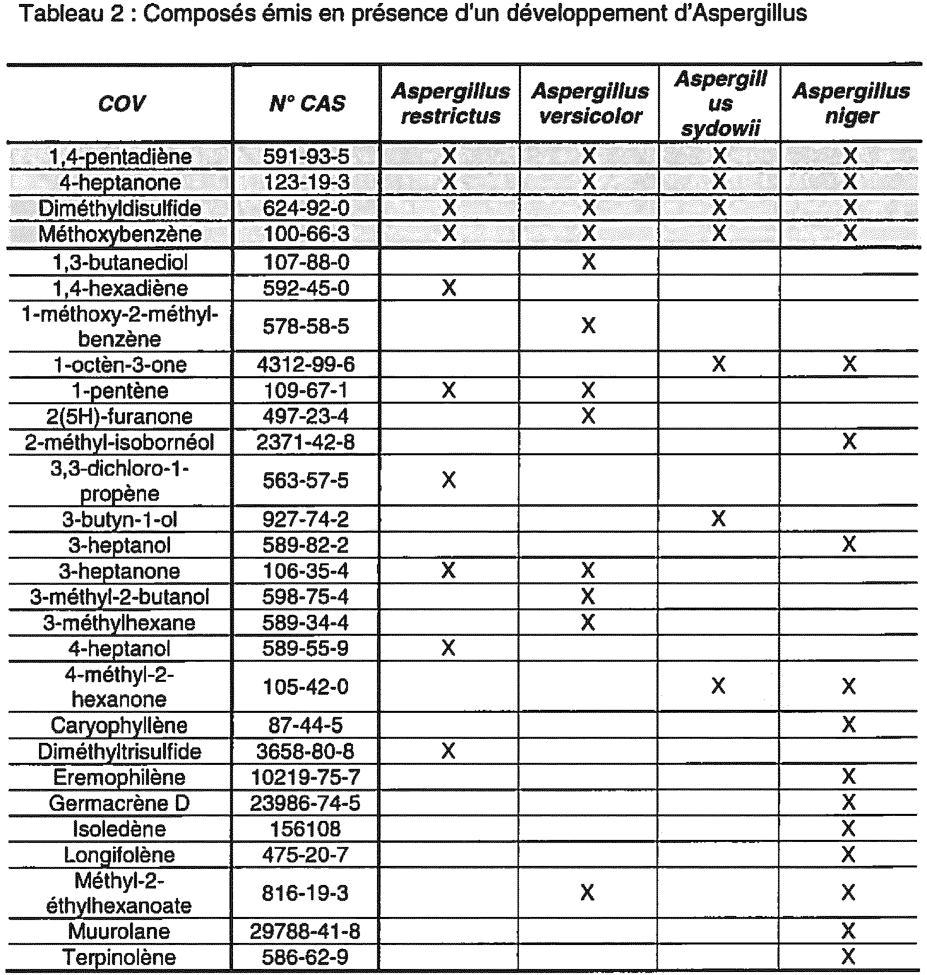

Le tableau 2 énumère les COV émis en présence des différentes souches d'Aspergillus. Les prélèvements et l'analyse de ces COV sont réalisés après 7 jours d'incubation des souches à 25°C. Les croix indiquent l'identification du COV à partir de la croissance de l'espèce sur au moins un support de croissance.

Comme on peut le voir sur le tableau 2, les quatre premiers COV sont des marqueurs de quatre espèces d'aspergillus, tandis que les autres COV sont marqueurs d'une ou deux espèces d'aspergillus.

Ainsi, cette liste de composés peut être scindée en 2 groupes :
Les composés émis par l'ensemble des espèces aspergillaires testées (groupe 1 surligné en gris dans le tableau).
Les composés émis par au moins une et au plus trois des quatre espèces aspergillaires testées (groupe 2).

D'un point de vue pratique, après détermination de la présence d'un développement fongique, par exemple par l'indice de contamination fongique, la recherche des cibles spécifiques listées dans le Tableau 2 permet d'alerter sur un développement probable d'espèces aspergillaires. En effet, la présence d'au moins une de ces cibles indique la présence probable d'un développement d'espèces aspergillaires.

Le nombre de traceurs identifiés est corrélé à la probabilité de présence d'un développement d'Aspergillus. L'absence de composés du groupe 1 diminue cette probabilité.

D'autres molécules cibles peuvent être identifiées. De manière générale, des molécules cibles de ce type peuvent consister en tout COV en lien avec les schémas métaboliques aspergillaire, c'est-à-dire un COV produit par une souche d'Aspergillus pendant le métabolisme aspergillaire.

Ainsi, la détermination d'une empreinte de contamination aspergillaire basée sur la détection de COVm chimiques spécifiques permettant de compléter les indices de contamination fongique déjà développés dans la demande WO 2008/125770, en fournissant des critères clairs et fiables pour les décisions concernant par exemple l'occupation et la rénovation de bâtiments contaminés.

Dans la mise en oeuvre du procédé selon une variante préférée, on réalise, successivement les étapes de :
a) prélèvement d'un échantillon d'air dans un environnement intérieur, par exemple à proximité de zones suspectées d'êtres contaminées ;
b) détection de COV dans l'échantillon, qui comprend une détection de la présence ou de l'absence de certains COV prédéterminés issus du métabolisme fongique, ces COV prédéterminés comprenant au moins un COV de chacune des trois catégories de COV suivantes :
   (1) les COV qui sont émis indépendamment de l'espèce fongique et de son support et qui ne sont émis que par des espèces fongiques ;
   (2) les COV qui sont émis indépendamment de l'espèce fongique et du support, mais qui peuvent également avoir d'autres origines biologiques par COV ayant « d'autres origines biologiques », on entend notamment des COV émis par des espèces biologiques non fongiques ;
   (3) les COV qui sont émis en fonction de l'espèce fongique et/ou de son support ;
c) calcul d'un indice chimique de contamination fongique en fonction respectivement de la présence et de l'absence des COV prédéfinis, issus du métabolisme fongique, conformément au procédé décrit dans la demande WO 2008/125770, pour déterminer s'il y a une contamination fongique ;
Ensuite, pour déterminer s'il y'a une contamination aspergillaire, on réalise en outre les étapes de :
d) recherche d'au moins une molécule cible, qui est un COV issue d'une métabolisme aspergillaire, en particulier au moins une molécule cible sélectionnée dans le groupe comprenant le : 1,4-pentadiène, 4-heptanone, Diméthyldisulfide, Méthoxybenzène, 1,3-butanediol, 1,4-hexadiène, 1-méthoxy-2-méthyl-benzène, 1-octèn-3-one, 1-pentène, 2(5H)-furanone, 2-méthyl-isoboméol, 3,3-dichloro-1-propène, 3-butyn-1-ol, 3-heptanol, 3-heptanone, 3-méthyl-2-butanol, 3-méthylhexane, 4-heptanol, 4-méthyl-2-hexanone, Caryophyllène, Diméthyltrisulfide, Eremophilène, Germacrène D, Isoledène, Longifolène, Méthyl-2-éthylhexanoate, Muurolane, Terpinolène ; et de préférence,
e) recherche d'une empreinte chimique comprenant au moins deux desdites molécules cibles.

De façon intéressante, nouvelle et inventive, les résultats issus des étapes d) et e) permettent de déterminer avec précision, clarté et fiabilité s'il y a ou non un risque de contamination aspergillaire.

Ce mode de réalisation aboutit bien entendu à des résultats plus complets que ceux de l'art antérieur, en ce qu'on arrive non seulement à conclure à une contamination fongique sans aucun signe visible de développement fongique, mais en plus on arrive à déterminer un développement aspergillaire de manière précise et fiable.

Dans une autre variante de l'invention, on peut aussi rechercher des zones de contamination fongique ; puis prélever un échantillon d'air à proximité de ces zones de contamination fongique avant de rechercher ladite ou lesdites molécules cibles évoquées ci-dessus.

Une telle recherche de zones de contamination fongique peut se faire par exemple à l'oeil nu, par des analyses en microscopie ou par des tests microbiologiques ou biochimiques.

Le prélèvement de l'échantillon d'air est par exemple réalisé par échantillonnage diffusif sur un adsorbant solide de type carbographe 4. La détection est par exemple réalisée par chromatographie en phase gazeuse suivie de spectrométrie de masse (GC/MS). D'autres méthodes de détection peuvent être utilisées.

## Revendications

1. Procédé de détermination d'un risque de contamination aspergillaire dans un environnement intérieur comprenant les étapes de :
(a) prélèvement d'un échantillon d'air dans l'environnement intérieur, puis
(b) détection de Composés Organiques Volatiles microbiens (COVm) dans l'échantillon, **caractérisé en ce que** l'étape (b) comprend une recherche d'une empreinte chimique comprenant au moins une molécule cible qui est un COVm, associé à un métabolisme aspergillaire, ladite molécule cible étant sélectionnée parmi les COVm suivants : 1,4-pentadiène, 4-heptanone, Diméthyldisulfide, Méthoxybenzène, 1,3-butanediol, 1,4-hexadiène, 1-méthoxy-2-méthyl-benzène, 1-octèn-3-one, 1-pentène, 2(5H)-furanone, 2-méthyl-isoboméol, 3,3-dichloro-1-propène, 3-butyn-1-ol, 3-heptanol, 3-heptanone, 3-méthyl-2-butanol, 3-méthylhexane, 4-heptanol, 4-méthyl-2-hexanone, Caryophyllène, Diméthyltrisulfide, Eremophilène, Germacrène D, Isoledène, Longifolène, Méthyl-2-éthylhexanoate, Muurolane, Terpinolène.

2. Procédé de détermination d'un risque de contamination aspergillaire dans un environnement intérieur selon la revendication 1, **caractérisé en ce que** l'étape (b) comprend les sous-étapes suivantes avant la recherche d'une empreinte chimique comprenant au moins une molécule cible qui est un COVm, associé à un métabolisme aspergillaire :
- détection de la présence ou de l'absence de certains Composés Organiques Volatiles (COV) prédéterminés issus du métabolisme fongique, ces COV prédéterminés comprenant au moins un COV de chacune des trois catégories de COV suivantes :
(1) les COV qui sont émis indépendamment de l'espèce fongique et de son support et qui ne sont émis que par des espèces fongiques ;
(2) les COV qui sont émis indépendamment de l'espèce fongique et du support, et qui sont émis par des espèces biologiques non fongiques;
(3) les COV qui sont émis en fonction de l'espèce fongique et/ou de son support ;
- calcul d'un indice chimique de contamination fongique en fonction respectivement de la présence et de l'absence des COV prédéfinis issus du métabolisme fongique.

3. Procédé selon la revendication 1 ou 2, **caractérisé en ce que** l'empreinte chimique est spécifique d'au moins une espèce d'aspergillus choisie parmi *Aspergillus restrictus, Aspergillus versicolor, Aspergillus sydowii, Aspergillus niger.*

4. Procédé selon l'une des revendications 1 à 3, **caractérisé en ce que** l'empreinte chimique comprend au moins deux desdites molécules cibles.

5. Procédé selon l'une des revendications 1 à 4, **caractérisé en ce que** l'empreinte chimique comprend toutes lesdites molécules cibles.

6. Procédé selon l'une des revendications 1 à 5, **caractérisé en ce que** ladite molécule cible est spécifique d'une ou deux souches d'Aspergillus.

7. Procédé selon la revendication 6, **caractérisé en ce que** ladite molécule cible est sélectionnée dans le groupe comprenant le : 1,3-butanediol, 1,4-hexadiène, 1-méthoxy-2-méthyl-benzène, 1-octèn-3-one, 1-pentène, 2(5H)-furanone, 2-méthyl-isobornéol, 3,3-dichloro-1-propène, 3-butyn-1-ol, 3-heptanol, 3-heptanone, 3-méthyl-2-butanol, 3-méthylhexane, 4-heptanol, 4-méthyl-2-hexanone, Caryophyllène, Diméthyltrisulfide, Eremophilène, Germacrène D, Isoledène, Longifolène, Méthyl-2-éthylhexanoate, Muurolane, Terpinolène.

8. Procédé selon l'une des revendications 1 à 7, **caractérisé en ce que** ladite molécule cible est spécifique de plus de deux souches d'Aspergillus, et est de préférence sélectionnée dans le groupe comprenant le : 1,4-pentadiène, 4-heptanone, Diméthyldisulfide, Méthoxybenzène.

9. Procédé selon l'une des revendications 1 à 8, comprenant une étape de recherche de zones de contamination fongique réalisée avant l'étape (a).

## Patentansprüche

1. Verfahren zur Bestimmung einer Kontaminie-rungsgefahr durch Aspergillus in einer Innenumgebung, umfassend die Schritte:
(a) Entnahme einer Luftprobe in einer Innenumgebung, dann
(b) Detektion von mikrobiellen flüchtigen organischen Substanzen (COVm) in der Probe, **dadurch gekennzeichnet, dass** der Schritt (b) eine Suche eines chemischen Fingerabdrucks umfasst, umfassend wenigstens ein Zielmolekül, das ein COVm ist, das einem Aspergillus-Metabolismus zugeordnet ist, wobei das genannte Zielmolekül ausgewählt ist aus den folgenden COVm: 1,4-Pentadien, 4-Heptanon, Dimethyldisulfid, Methoxybenzen, 1,3-Butandiol, 1,4-Hexadien, 1-Methoxy-2-Methyl-Benzen, 1-Octen-3-on, 1-Penten, 2(5H)-Furanon, 2-Methyl-Isoborneol, 3,3-Dichlor-1-Propen, 3-Butyn-1-ol, 3-Heptanol, 3-Heptanon, 3-Methyl-2-Butanol, 3-Methylhexan, 4-Heptanol, 4-Methyl-2-Hexanon, Caryophyl-len, Dimethyltrisulfid, Eremophilen, Germacren D, Isoleden, Longifolen, Methyl-2-Ethylhexanoat, Muurolan, Terpinolen.

2. Verfahren zur Bestimmung einer Kontaminierungsgefahr durch Aspergillus in einer Innenumgebung gemäß Anspruch 1, **dadurch gekennzeichnet, dass** der Schritt (b) die folgenden Teilschritte vor der Suche eines chemischen Fingerabdrucks umfasst, umfassend wenigstens ein Zielmolekül, das ein COVm ist, das einem Aspergillus-Metabolismus zugeordnet ist:
- Detektion des Vorhandenseins oder des Fehlens von bestimmten vorbestimmten, aus dem Pilzmetabolismus stammenden flüchtigen organischen Substanzen (COV), wobei diese vorbestimmten COV wenigstens eine COV jeder der drei folgenden COV-Kategorien umfassen:
(1) die COV, die unabhängig von der Pilzart und deren Träger ausgegeben werden und die nur von Pilzarten ausgegeben werden;
(2) die COV, die unabhängig von der Pilzart und dem Träger ausgegeben werden und die von biologischen Nicht-Pilzarten ausgegeben werden;
(3) die COV, die in Abhängigkeit von der Pilzart und / oder ihrem Träger ausgegeben werden;
- Berechnung eines chemischen Pilzkontaminierungsindexes in Abhängigkeit jeweils von dem Vorhandensein und dem Fehlen der vordefinierten, aus dem Pilzmetabolismus stammenden COV.

3. Verfahren gemäß Anspruch 1 oder 2, **dadurch gekennzeichnet, dass** der chemische Fingerabdruck spezifisch für wenigstens eine Aspergillus-Art ist, die ausgewählt ist aus Aspergillus restrictus, Aspergillus versicolor, Aspergillus sydowii, Aspergillus niger.

4. Verfahren gemäß einem der Ansprüche 1 bis 3, **dadurch gekennzeichnet, dass** der chemische Fingerabdruck wenigstens zwei der genannten Zielmoleküle umfasst.

5. Verfahren gemäß einem der Ansprüche 1 bis 4, **dadurch gekennzeichnet, dass** der chemische Fingerabdruck alle die genannten Zielmoleküle umfasst.

6. Verfahren gemäß einem der Ansprüche 1 bis 5, **dadurch gekennzeichnet, dass** das genannte Zielmolekül für einen oder zwei Aspergillus-Stamm / -Stämme spezifisch ist.

7. Verfahren gemäß Anspruch 6, **dadurch gekennzeichnet, dass** das genannte Zielmolekül ausgewählt ist aus der Gruppe umfassend: 1,3-Butandiol, 1,4-Hexadien, 1-Methoxy-2-Methyl-Benzen, 1-Octen-3-on, 1-Penten, 2(5H)-Furanom, 2-Methyl-Isoborneol, 3,3-Dichlor-1Propen, 3-Butyn-1-ol, 3-Heptanol, 3-Heptanon, 3-Methyl-2-Butanol, 3-Methylhexan, 4-Heptanol, 4-Methyl-2Hexanon, Caryophyllen, Dimethyltrisulfid, Eremophilen, Germacren D, Isoleden, Longifolen, Methyl-2Ethylhexanoat, Muurolan, Terpinolen.

8. Verfahren gemäß einem der Ansprüche 1 bis 7, **dadurch gekennzeichnet, dass** das genannte Zielmolekül für mehr als zwei Aspergillus-Arten spezifisch ist und bevorzugt aus der Gruppe ausgewählt ist, umfassend: 1,4-Pentadien, 4-Heptanon, Dimethyldisulfid, Methoxybenzen.

9. Verfahren gemäß einem der Ansprüche 1 bis 8, umfassend einen Schritt zur Suche von Pilzkontaminierungsbereichen, der vor dem Schritt (a) realisiert wird.

## Claims

1. A method to determine a risk of Aspergillus contamination in an indoor environment comprising the following steps:
a) collection of a sample of the air in the indoor environment, and
b) detection of microbial volatile organic compounds (MVOC) in the sample, **characterised in that** step b) comprises a search for a chemical fingerprint comprising at least one target molecule which is a MVOC, associated with an Apsergillus metabolism, said target molecule is selected from among the following MVOC: 1,4-pentadiene, 4-heptanone, Dimethyldisulfide, Methoxybenzene, 1-3-butanediol, 1,4-hexadiene, 1methoxy-2-methyl-benzene, 1-octen-3-one, 1-pentene, 2(5H)-furanone, 2-methyl-isoborneol, 3,3-dichloro-1-propene, 3-butyn-1-ol, 3-heptanol, 3-heptanone, 3-methyl-2-butanol, 3-methylhexane, 4-heptanol, 4-methyl-2-hexanone, Caryophyllene, Dimethyltrisulfide, Eremophilene, Germacrene D, Isoledene, Longifolene, Methyl-2-ethylhexanoate, Muurolane, Terpinolene.

2. A method to determine a risk of Aspergillus contamination in an indoor environment according to claim 1, **characterised in that** step b) comprises the following sub-steps before the search for a chemical fingerprint comprising at least one target molecule which is an MVOC, associated with an Aspergillus metabolism:
- detection of the presence or absence of certain predetermined volatile organic compounds (VOC) from the fungal metabolism, these predetermined VOCs comprising at least one VOC from each of the following three VOCs:
(1) VOCs that are released independently of the fungal species and its support and are only issued by fungal species;
(2) VOCs that are released independently of the fungal species and the support, and which are issued by non-fungal biological species;
(3) VOCs that are released according to the fungal species and/or its support
- calculation of a chemical index of fungal contamination according to the presence and absence, respectively, of predefined VOCs derived from the fungal metabolism.

3. Method according to claim 1 or 2, **characterised in that** the chemical fingerprint is specific for at least one Aspergillus species selected from among *Aspergillus restrictus, Aspergillus versicolor, Aspergillus sydowii, Aspergillus niger.*

4. Method according to one of claims 1 to 3, **characterised in** the chemical fingerprint comprises at least two of the said target molecules.

5. Method according to one of claims 1 to 4, **characterised in that** the chemical fingerprint comprises all of said target molecules.

6. Method according to one of claims 1 to 5, **characterised in that** said target molecule is specific for one or two Aspergillus strains.

7. Method according to claim 6, **characterised in that** said target molecule is selected from the group comprising: 1,3-butanediol, 1,4-hexadiene, 1-methoxy-2-methyl-benzene, 1-octen-3-one, 1-pentene, 2(5H)-furanone, 2-methyl-isoborneol, 3,3-dichloro-1-propene, 3-butyn-1-ol, 3-heptanol, 3-heptanone, 3-methyl-2-butanol, 3-methylhexane, 4-heptanol, 4-methyl-2-hexanone, Caryophyllene, Dimethyltrisulfide, Eremophilene, Germacrene D, Isoledene, Longifolene, Methyl-2-ethylhexanoate, Muurolane, Terminolene.

8. Method according to one of claims 1 to 7, **characterised in that** said target molecule is specific for more than two strains of Aspergillus, and is preferably selected from the group comprising: 1,4-pentadiene, 4-heptanone, Dimethyldisulfide, Methoxybenzene.

9. Method according to one of claims 1 to 8, comprising a search for zones of fungal contamination performed before step (a).
